# EUROPÄISCHE PATENTSCHRIFT

(11) **EP 1 311 305 B1**
(45) Veröffentlichungstag und Bekanntmachung des Hinweises auf die Patenterteilung: **11.08.2004**
(21) Anmeldenummer: 01949364.2
(22) Anmeldetag: 29.05.2001
(51) Int. Cl.: A61L 2/26

(54) **SIEB, INSBESONDERE STERILISATIONSSIEB**
SCREEN, PARTICULARLY A STERILISATION SCREEN
TAMIS, EN PARTICULIER TAMIS DE STERILISATION

(30) Priorität: 26.08.2000 DE 10042083
(43) Veröffentlichungstag der Anmeldung: 21.05.2003
(73) Patentinhaber: Metallverarbeitung Kögel GmbH, 75038 Oberderdingen (DE)
(72) Erfinder: KÖGEL, Rolf-Dieter, 75038 Oberderdingen (DE); SERVAY, Gerd, 75057 Kürnbach (DE)
(74) Vertreter: Bulling, Alexander, Dr.
(86) Internationale Anmeldenummer: PCT/EP2001/006078
(87) Internationale Veröffentlichungsnummer: WO 2002/017976

(56) Entgegenhaltungen:
- DE-U- 8 503 693
- DE-U- 29 710 171
- DE-U- 29 713 915
- US-A- 5 540 901

## Beschreibung

Die Erfindung betrifft ein Sieb, insbesondere ein Sterilisationssieb, mit einem Boden und mit an dem Boden angeordneten Seitenwänden, wobei der Boden und/oder die Seitenwände wenigstens abschnittsweise aus Siebmaterial gebildet sind.

Derartige Siebe finden beispielsweise in Krankenhäusern, in Kliniken, Labors oder ähnlichen Einrichtungen zur Sterilisation von entsprechenden Gütern Verwendung. Darüber hinaus können die Siebe zur Lagerung, zum Transport, zur Organisation odgl. von den Gütern eingesetzt werden. Die Sterilisationsgüter werden hierbei in ein solches Sieb gelegt. In einem nächsten Arbeitsschritt wird das Sieb mit einem Vlies umwickelt, um ein Herausfallen der Sterilisationsgüter aus dem Sieb zu vermeiden. Ferner wird das umwickelte Sieb in einen Blechbehälter gegeben und mit dem Blechbehälter in einen Sterilisator gegeben bzw. in ein Sterilisationsbad getaucht, in welchem der Sterilisationsvorgang stattfindet.

Die Handhabung der Siebe, und insbesondere der mit Vlies umwickelten Siebe, hat sich als umständlich und als problematisch herausgestellt. Nach dem Sterilisationsvorgang verbleiben die Güter in dem Sieb bzw. in dem Blechbehälter. Ein schnelles Trocknen der Güter kann nicht stattfinden. Außerdem sind die Siebe nach ihrem verwendungsgemäßen Gebrauch sperrig handzuhaben und zu transportieren.

Aus der DE 297 10 171 U1 ist ein Sterilisations-Siebkorb bekannt geworden, der an der Oberkante der Seitenwände lediglich ein Rahmenelement in Form eines Verstärkungsdrahtes aufweist. An dem Rahmenelement sind nach seitlich außen über die Seitenwände und nach oben ragende Verbundführungen vorgesehen. Bei diesem vorbekannten Sterilisations-Siebkorb sind die Verbundführungen ausschließlich an den Oberkanten der sich jeweils gegenüberliegen Stirn- und Längswänden vorgesehen. Die Stirn- und Längswände liegen jeweils zwischen Eckwandbereichen, an denen keine Verbundführungen vorgesehen sind. Um ein sicheres Stapeln von mehreren Sterilisations-Siebkörben zu ermöglichen, sind bei diesem Stand der Technik Verbundführungen sowohl an den Stirnwänden als auch an den Längswänden vorgesehen. Diese seitlich abstehenden und nach oben ragenden Verbundführungen machen den Sterilisations-Siebkorb zum einen in der Herstellung aufwändig und zum anderen verschlechtern sie dessen Handhabung.

In der DE 85 03 693 U wird eine Siebschale beschrieben, die an der Oberkante der Seitenwände ein Rahmenelement in Form einer oberen Rahmenstrebe aufweist. An der Unterseite der Siebschale sind eine untere Rahmenstrebe und parallel zu der unteren Rahmenstrebe verlaufende Bügel angeordnet. Durch die Bügel wird ein Stapeln von mehreren Siebschalen ermöglicht.

Der vorliegenden Erfindung liegt die Aufgabe zugrunde, ein Sieb, insbesondere ein Sterilisationssieb vorzuschlagen, welches einfach handhabbar ist und dennoch ein sicheres Sterilisieren der Sterilisationsgüter gewährleistet. Insbesondere soll ein sicherer Schutz der Sterilisationsgüter während des Sterilisationsvorganges gewährleistet und die Handhabbarkeit der Siebe vereinfacht werden. Ein rasches Trocknen der Güter nach dem Sterilisationsvorgang soll ferner ermöglicht werden. Die Sterilisationsgüter sollen auch während des Transports in beispielsweise den Operationsraum eines Krankenhauses in den Sieben sicher geschützt werden können.

Diese Aufgabe wird durch ein Sieb mit den Merkmalen des Anspruchs 1 gelöst.

Die Erhebungen und die Vertiefungen sind komplementär ausgebildet, wodurch ein formschlüssiges Aufliegen gewährleistet wird und ein Herunterrutschen oder Herunterschieben des oberen Siebes von dem unteren nicht oder nur schwer möglich ist. Die Erfindung hat dabei den Vorteil, dass aufgrund der entsprechenden Ausbildung der Rahmenelemente mit Erhebungen und Vertiefungen zwei Siebe der Art aufeinander gelegt werden, dass ein von den beiden Sieben umschlossener Raum gebildet wird. Werden zwei aufeinander liegende Siebe in den Sterilisator gebracht bzw. in das Sterilisationsbad getaucht, so ist durch den von den beiden Sieben umschlossenen Raum ein sicherer Schutz der Sterilisationsgüter gegeben. Dabei werden die Siebe mit einem Vlies umwickelt und können unmittelbar - ohne Verwendung eines Blechbehälters - in den Sterilisator gegeben werden. Aufgrund der Stabilität der Siebe kann auf einen Blechbehälter, wie er gemäß dem bekannten Stand der Technik erforderlich ist, verzichtet werden. Dies hat auch zur Folge, dass die Güter unmittelbar nach dem Sterilisationsvorgang schnell abtrocknen können. Vorteilhafterweise wird die Handhabung des Siebes, der Sterilisationsgüter und des gesamten Sterilisationsvorganges wesentlich vereinfacht und verbessert.

Erfindungsgemäß kann vorgesehen sein, dass sich die Vertiefungen und Erhebungen über die Eckbereiche erstrecken. Die Eckbereiche liegen dabei insbesondere zwischen den jeweils an die Eckbereiche angrenzenden Stirn- und Längswänden. Bei einem bündigen bzw. formschlüssigen Aufeinanderliegen der entsprechenden Rahmenelemente können hierbei mit einer geringen Anzahl von Vertiefungen und Erhebungen aufgrund des Formschlusses horizontal auf die Siebe wirkende Kräfte gut übertragen werden. Damit wird mit relativ wenigen Vertiefungen und Erhebungen ein sicheres und stabiles Aufeinanderliegen der beiden Siebe erreicht.

Bei einer besonders bevorzugten Ausgestaltung der Erfindung ist vorgesehen, dass an den jeweils gegenüberliegenden, also nicht benachbarten, Eckbereichen entweder Erhebungen oder Vertiefungen vorhanden sind. Bei einem Sieb, welches beispielsweise vier Seitenwandungen und damit vier Eckbereiche aufweist, kann hierdurch vorteilhafterweise erreicht werden, dass ein Aufeinanderliegen der Siebe in zwei verschiedenen Positionen möglich ist. Die eine Position wird dann erreicht, wenn die Erhebungen des einen Siebes in den komplementär dazu ausgebildeten Vertiefungen des anderen Siebes angeordnet sind. Weiterhin kann beispielsweise das obere Sieb um eine vertikale Achse um 180° gedreht werden. Auch in dieser Position ist ein formschlüssiges Aufliegen des oberen Siebes auf dem unteren möglich. Ein falsches, d.h. nicht passendes Auflegen des zweiten Siebes auf das erste wird damit ausgeschlossen.

Erfindungsgemäß ist bei Vorsehen von zwei Rahmenelementen an der Oberkante der Seitenwände denkbar, dass lediglich eines der beiden Rahmenelemente, insbesondere das äußere Rahmenelement, Erhebungen oder Vertiefungen aufweist und dass das andere Rahmenelement, insbesondere das innere Rahmenelement, keine Erhebungen oder Vertiefungen aufweist. Dies hat den Vorteil, dass lediglich eines der beiden Rahmenelemente zur Realisierung der Erhebungen oder Vertiefungen zu bearbeiten ist. Bei zwei aufeinander liegenden, einen Aufnahmeraum für zu sterilisierende Güter bildenden Sieben stützen sich zur Aufnahme von horizontal wirkenden Kräften oder Kraftkomponenten die Erhebungen und Vertiefungen des einen Rahmenelements des einen Siebes an den nicht in vertikale Richtung verformten, unmittelbar an den Erhebungen und Verformungen anliegenden Abschnitten des anderen Rahmenelements des anderen Siebes ab.

Um zwei aufeinander liegende Siebe, insbesondere während des Sterilisationsvorganges, funktionssicher miteinander zu verbinden, kann erfindungsgemäß vorgesehen sein, dass die Siebe Verbindungsmittel aufweisen, die zum Verbinden der beiden aufeinander liegenden Siebe vorgesehen sind. Derartige Verbindungsmittel können beispielsweise Schnapphaken, Hakenelemente, Krallen oder dergleichen sein.

Die Rahmenelemente sind gemäß Anspruch 1 Drahtelemente, die um die Oberkanten der Seitenwände laufen. Derartige Drahtelemente, die vorzugsweise einen kreisrunden Querschnitt aufweisen, lassen sich auf einfache und kostengünstige Art und Weise fertigen.

Bei einer weiteren Ausgestaltungsform der Erfindung ist vorgesehen, dass sowohl der Boden als auch die Seitenwände von einem Drahtgeflecht gebildet werden, wobei die freien Enden der das Drahtgeflecht bildenden Drähte zwischen den beiden Rahmenelementen an der Oberkante der jeweiligen Seitenwände enden. Hierdurch wird aufgrund des einstückig ausgebildeten Drahtgeflechts ein sehr günstiges und vorteilhaftes Siebmaterial gebildet. Dadurch, dass die freien Enden der das Drahtgeflecht bildenden Drähte zwischen den beiden Rahmenelementen enden, sind diese nicht zugänglich. Eine Verletzungsgefahr an den Enden ist hierbei ausgeschlossen. In den Bereichen, in denen das eine Rahmenelement Erhebungen bzw. Vertiefungen aufweist kann vorgesehen sein, dass die freien Enden der Drähte an lediglich nur einem Rahmenelement anliegen, wobei diese freien Enden dann beispielsweise abgefeilt bzw. abgeschliffen werden können, um eine Verletzungsgefahr auch hier auszuschließen.

Um das Sieb in sich zu stabilisieren, kann vorteilhafterweise an den Seitenwänden ein weitgehend parallel zu dem Boden verlaufendes Mittelrahmenelement vorgesehen sein.

Weiterhin kann bei einer Ausgestaltung der Erfindung das Mittelrahmenelement insbesondere zwei gegenüberliegende Ausformungen aufweisen, die als Griffe Verwendung finden können. Hierdurch wird die Handhabbarkeit des Siebes weiterhin vereinfacht.

Um eine Platz sparende Lagerung erfindungsgemäßer Siebe zu ermöglichen, können die Siebe der Art ausgebildet sein, dass sie ineinander stapelbar sind. Hierbei liegen vorteilhafterweise die Ausformungen des Mittelrahmenelements des oberen Siebes auf der Oberseite der beiden Rahmenelemente des unteren Siebes auf. Durch diese definierte Auflage wird ein Verklemmen und Verkanten der ineinander gestapelten Siebe unterbunden.

Weiterhin kann erfindungsgemäß vorgesehen sein, dass das Sieb ein auf der Unterseite des Bodens angeordnetes Bodenrahmenelement aufweist, welches der der Oberkante der Seitenwände abgewandte Ausformungen aufweist. Diese Ausformungen dienen dabei als Füße, welche bei einem Stellen des Siebes auf einen Untergrund auf dem Untergrund zum Aufliegen kommen. Damit kommt das Siebmaterial mit dem Untergrund nicht Berührung, wodurch Verschmutzungen des Siebmaterials vermieden werden.

Vorteilhafterweise sind derartige Ausformungen an Querverstrebungen angeordnet, welche das Bodenrahmenelement aufweist.

Bei einer weiteren Ausgestaltungsform der Erfindung sind wenigstens zwei Siebe quer zueinander aufeinander stapelbar, wobei das Bodenrahmenelement des oberen Siebes auf den Rahmenelementen des unteren Siebes aufliegt und die Ausformungen an dem Bodenrahmenelement des oberen Siebes eine Verschiebbarkeit des oberen Siebes in Querrichtung verhindern. Hierdurch wird eine besonders gunstige Stapelbarkeit der Siebe erreicht, auch wenn in dem Sieb bzw. in den Sieben Güter vorhanden sind.

Vorteilhafterweise ist das erfindungsgemäße Sieb vollständig aus Edelstahl. Edelstahl ist insbesondere auch gegenüber dem Sterilisationsbad korrosionsunanfällig.

Weitere vorteilhafte Ausgestaltungen und Einzelheiten der Erfindung sind der folgenden Beschreibung zu entnehmen, in der die Erfindung anhand des in der Zeichnung dargestellten Ausführungsbeispiels näher beschrieben und erläutert ist.
- Figur 1: ein erfindungsgemäßes Sieb in perspektivischer Darstellung,
- Figur 2 und Figur 3: zwei Ansichten zweier Eckbereiche des Siebes gemäß Fig. 1 in schematischer Darstellung,
- Figur 4: die Ansicht gemäß Fig. 3 in perspektivischer Darstellung,
- Figur 5: zwei aufeinander liegende erfindungsgemäße Siebe,
- Figur 6: ein Ausschnitt zweier ineinander gestapelter Siebe,
- Figur 7: zwei quer zueinander, aufeinander gestapelte Siebe.

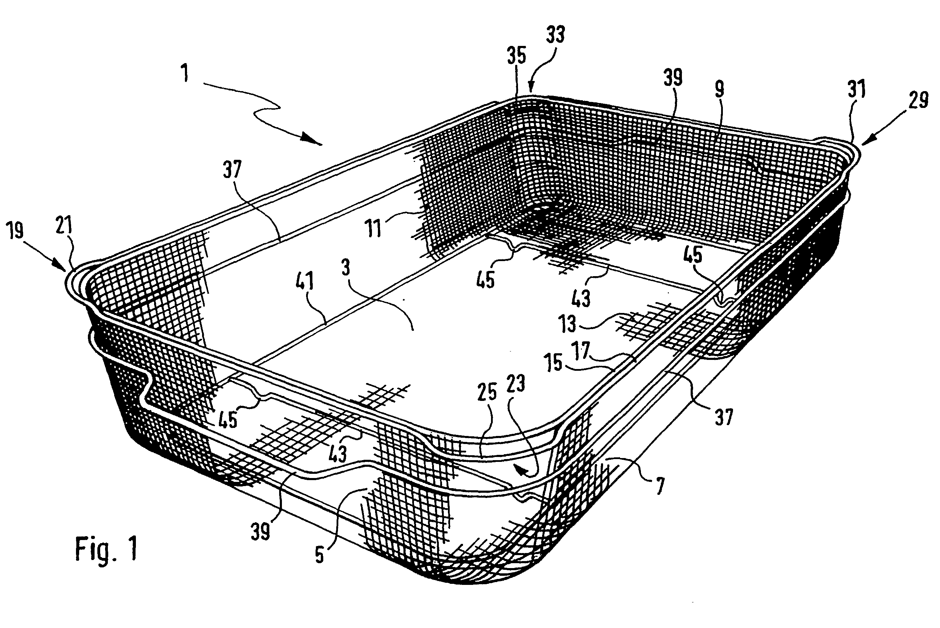

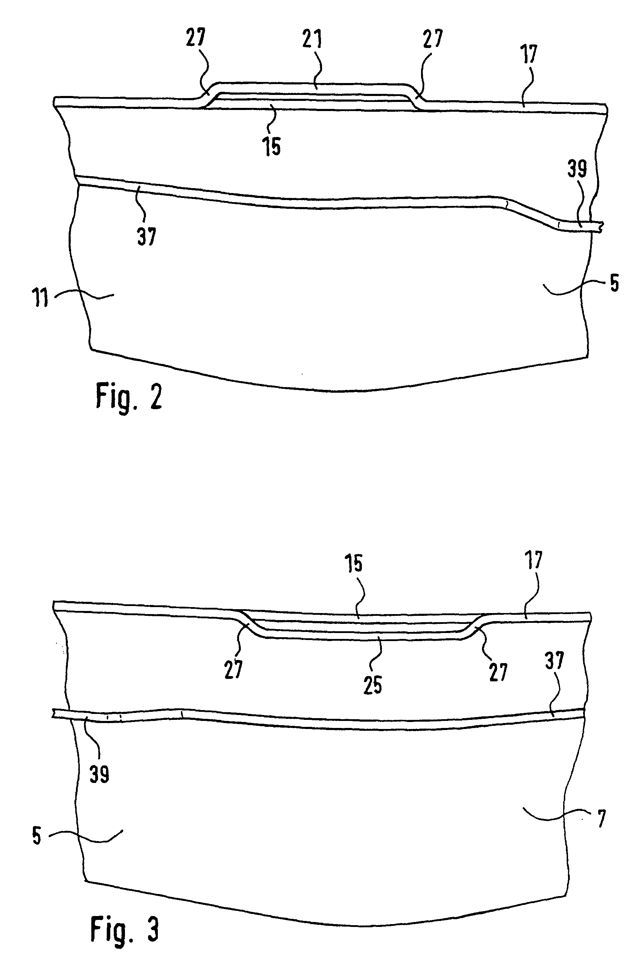

In der Figur 1 ist ein erfindungsgemäßes Sterilisationssieb 1 dargestellt, welches einen Boden 3 sowie vier an dem Boden 3 angeordnete Seitenwände 5, 7, 9 und 11 aufweist. Der Boden 3 sowie die Seitenwände 5, 7, 9 und 11 sind hierbei von einem Drahtgeflecht 13 gebildet. Das Drahtgeflecht 13 ist in der Figur 1 ausschnittsweise schematisch angedeutet. In den Figuren 2, 3, 5 bis 7 ist das Drahtgeflecht zur besseren Veranschaulichung des erfindungsgemäßen Siebes 1 nicht dargestellt. Lediglich die Konturen des Drahtgeflechts 13 sind angedeutet. Das in den Figuren dargestellte Sterilisationssieb 1 ist vorzugsweise aus Edelstahl, um ein Korrodieren des Siebmaterials auszuschließen.

Die Oberkanten der Seitenwände 5, 7, 9 und 11 bilden eine Öffnung, über welche Gegenstände in das Sieb 1 eingelegt bzw. aus dem Sieb 1 herausgenommen werden können.

Das in der Figur 1 dargestellte Sieb 1 weist weiterhin an den Oberkanten der Seitenwände zwei weitgehend parallel nebeneinander verlaufende Rahmenelemente 15 und 17 auf, die aus Drahtelementen gebildet sind und um die Oberkanten der Seitenwände 5, 7, 9 und 11 laufen. In den Längs- und Querseiten der Oberkante des Siebes 1 liegen die beiden Rahmenelemente 15 und 17 weitgehend in einer zu dem Boden 3 parallelen Ebene.

In den Eckbereichen weist das Rahmenelement 17 Vertiefungen bzw. Erhöhungen auf. Das innenliegende Rahmenelement 15 weist keinerlei Vertiefungen oder Erhebungen auf.

In der Figur 2, die den Eckbereich 19 gemäß Figur 1 in Seitenansicht darstellt, ist eine Erhebung 21 des Rahmenelements 17 deutlich zu erkennen. Das in der Ansicht nach Figur 2 hinter dem Rahmenelement 17 liegende Rahmenelement 15 weist keine derartige Erhebung auf.

In der Figur 3, in der der Eckbereich 23 des Siebes dargestellt ist, ist deutlich zu erkennen, dass das Rahmenelement 17 in diesem Bereich 23 eine Vertiefung 25 gegenüber dem Rahmenelement 15 aufweist.

Die Erhebung 21 als auch die Vertiefung 25 erstrecken sich jeweils um den gesamten jeweiligen Eckbereich 19 und 23. Die Erhöhung 21 bzw. die Vertiefung 25 ist so ausgebildet, dass sie, abgesehen von Übergangsbereichen 27, weitgehend parallel zu dem Rahmenelement 15 verlaufen. In den Übergangsbereichen 27 wurde das Rahmenelement 17 plastisch verformt, um die Erhebung 21 bzw. die Vertiefung 25 zu realisieren.

Der dem Eckbereich 19 in Figur 1 gegenüberliegende Eckbereich 29 weist eine der Erhebung 21 entsprechende Erhebung 31 auf. Der dem Eckbereich 23 gegenüberliegende Eckbereich 33 der Oberkante des Siebes 1 weist eine der Vertiefung 25 entsprechende Vertiefung 35 auf.

Das erfindungsgemäße Sieb 1 sieht weiterhin ein weitgehend parallel zu dem Boden 3 verlaufendes Mittelrahmenelement 37 vor. Das Mittelrahmenelement 37 ist als umlaufendes Drahtelement ausgebildet und an der Außenseite der Seitenwände 5, 7, 9 und 11 angeordnet. Ein derartiges Mittelrahmenelement 37 hat eine das Sieb 1 und insbesondere das Siebmaterial 13 verstärkende und stabilisierende Wirkung.

An dem Mittelrahmenelement 37 sind an den Stirnseiten des Siebes 1 zwei gegenüberliegende Ausformungen in Form von Griffen 39 angeordnet. Derartige Griffe 39 erleichtern die Handhabbarkeit des Siebes.

Das in den Figuren dargestellte erfindungsgemäße Sieb 1 weist weiterhin auf der Unterseite des Bodens 3 ein Bodenrahmenelement 41 auf, dass als umlaufender Draht ausgebildet ist. Um die Stabilität des Siebes 1 zu erhohen, sind an dem Bodenrahmenelement zwei Querverstrebungen 43 angeordnet. Die Querverstrebungen 43 sehen dabei in der dem Sieb 1 abgewandten Richtung ragende Ausformungen 45 vor, auf den das Sieb 1 auf einem nicht dargestellten Untergrund steht. Die Querverstrebungen 43 sind beispielsweise mit dem Bodenrahmenelement 41 verschweißt.

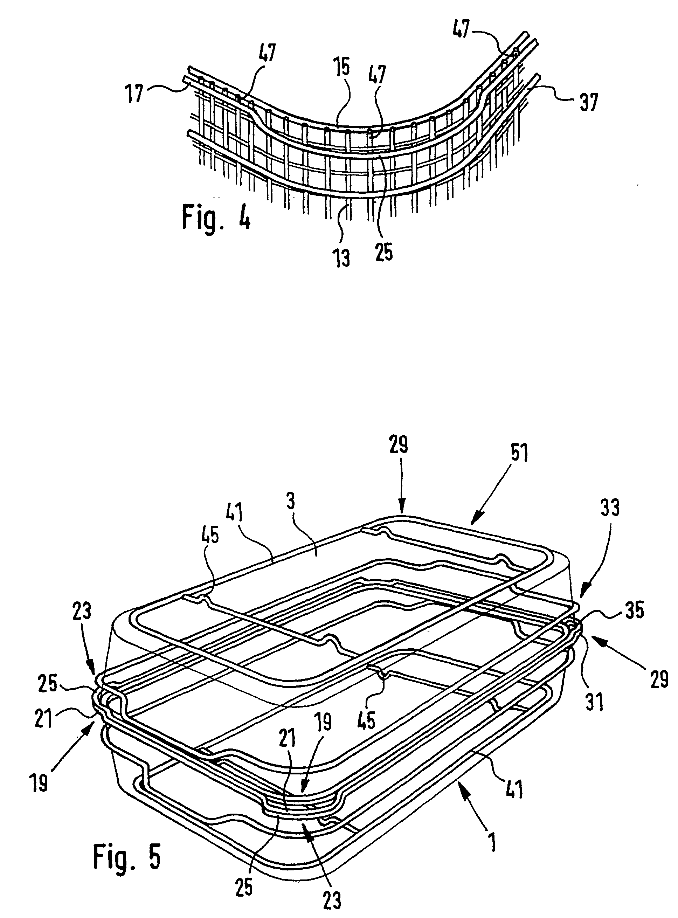

Um insbesondere Verletzungen an dem Sieb 1 zu vermeiden ist vorgesehen, dass die freien Enden, die in Figur 4 mit der Bezugsziffer 47 gekennzeichnet sind, zwischen den beiden Rahmenelementen 15 und 17 enden. Da sich das Drahtgeflecht 13, wie insbesondere aus Figur 1 deutlich hervorgeht, über die Seitenwände 5, 7, 9 und 11 als auch über den Boden 3 des Siebes 1 einstückig erstreckt, weist das Drahtgeflecht 13 lediglich an der Oberkante, also zwischen den beiden Rahmenelementen 15 und 17, freie Enden auf. Einer Verletzungsgefahr an diesen freien Enden 47 wird dadurch begegnet, dass die freien Enden 47 zwischen den beiden Rahmenelementen 15 und 17 enden. Die freien Enden 47 stehen also nicht über die beiden Rahmenelemente 15 und 17 heraus.

In den Eckbereichen 19, 23, 29 und 33 des Siebes 1 sind die freien Enden 47 lediglich am Rahmenelement 15 angeordnet. Um auch hier eine Verletzungsgefahr zu vermeiden, kann vorgesehen sein, dass die freien Enden 47 in diesem Bereich mit dem Rahmenelement 15 verlötet sind und anschließend entsprechend flach gefeilt werden.

Um eine dauerhafte und funktionssichere Anbindung des Drahtgeflechts 13 an die beiden Rahmenelemente 15 und 17 zu gewährleisten, kann vorgesehen sein, dass die freien Enden 47 des Drahtgeflechts 13 mit den beiden Rahmenelementen 15 und 17 verlötet oder verschweißt werden.

In der Figur 5 ist neben dem Sieb 1 ein weiteres Sieb 51, das mit dem Sieb 1 identisch ist, dargestellt. Die dem Sieb 1 entsprechenden Merkmale sind bei dem Sieb 51 mit den dem Sieb 1 entsprechenden Bezugsziffern versehen. Das Sieb 51 liegt mit nach oben gerichtetem Boden mit den Rahmenelementen 15 und 17 auf den Rahmenelementen 15 und 17 des Siebes 1 bündig und formschlüssig auf. Wie bereits erwähnt weisen lediglich jeweils die äußeren Rahmenelemente 17 der beiden Siebe 1 und 51 in den Eckbereichen die Vertiefungen und Erhebungen auf. Die beiden innenliegenden Rahmenelemente sind eben, d.h. in einer Ebene liegend ausgebildet. Hierbei kommt der Eckbereich 19 mit Erhebung 21 des Siebes 51 auf dem Eckbereich 23 mit der Vertiefung 25 des Siebes 1 zum Aufliegen. Entsprechend verhält es sich mit den anderen drei Eckbereichen 23, 29 und 33. Die Erhebungen 21 und 31 des Rahmenelements 17 des Siebes 1 sind hierbei komplementär zu den Vertiefungen 25 und 35 des Rahmenelements 17 des Siebes 51 ausgebildet. Aufgrund der sich über die Eckbereiche 19, 23 und 29 und 33 erstreckenden Erhebungen 21 und 31 und Vertiefungen 25 und 35 und des bündigen bzw. formschlüssigen Aufliegens der Rahmenelemente 15 und 17 der beiden Siebe 1 und 51 können horizontal wirkende Kräfte aufgenommen werden. Dabei stützen sich die Erhebungen 21 und 31 und Vertiefungen 25 und 35 des einen Rahmenelements 17 des einen Siebes 1 bzw. 51 an den eben ausgebildeten Eckbereichen des anderen Rahmenelements 15 des anderen Siebes 51 bzw. 1 ab. Damit ist ein sicheres Aufliegen der Rahmenelemente 15 und 17 des Siebes 51 auf den Rahmenelementen 15 und 17 des Siebes 1 bzw. des Siebes 51 auf dem Sieb 1 gewährleistet. Ein Herunterrutschen oder Heruntergleiten des in Figur 5 dargestellten Siebes 51 von dem Sieb 1 ist folglich ausgeschlossen.

Erfindungsgemäß können beim Sterilisieren in dem von den beiden Sieben 1 und 51 umschlossenen Raum vorhandene Sterilisationsgüter während des Sterilisationsvorganges nicht verloren gehen. Zum Sterilisieren der entsprechenden Güter wird das Sieb 1 mit darauf bündig aufliegendem Sieb 51, dessen Boden nach oben gerichtet ist, verwendet. Die beiden derartig aufeinander liegenden Siebe 1 und 51 werden mit den darin vorhandenen Sterilisationsgütern beispielsweise in ein Sterilisationsbad getaucht. Da auch während des Tauchvorganges die beiden Siebe 1 und 51, wie in Figur 5 dargestellt, aufeinander liegen, können keinerlei zu sterilisierende Güter verloren gehen.

Erfindungsgemäß kann auch vorgesehen sein, was allerdings in den Figuren nicht dargestellt ist, dass die beiden Siebe 1 und 51 in der in Figur 5 dargestellten Position mittels Verbindungselementen, beispielsweise Schnappverschlüssen oder Schnapphaken, miteinander verbunden werden. Damit wird zusätzlich gewährleistet, dass ein Lösen des auf dem Sieb 1 aufliegenden Siebes 51 während des Sterilisationvorganges ausgeschlossen wird.

Dadurch, dass die Vertiefungen 25 und 35 sowie die Erhebungen 21 und 31 an den gegenüberliegenden Eckbereichen der Siebe 1 und 51 vorhanden sind, kann das in Figur 5 dargestellte Sieb 51 um eine vertikale Achse um 180° gedreht werden, wobei es dennoch bündig zum Aufliegen auf dem Sieb 1 kommt. Damit ist ein "falsches" Aufsetzen des Siebes 51 auf das Sieb 1 ausgeschlossen.

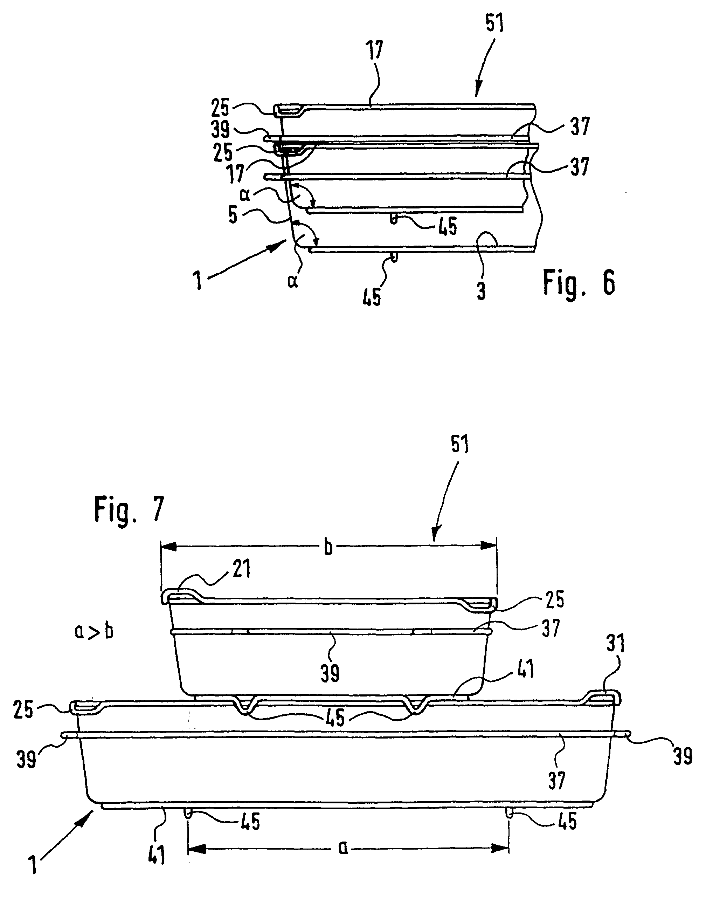

Figur 6 zeigt die Seitenansicht der beiden ineinander gestapelten Siebe 1 und 51. Um ein Ineinanderstapeln der Siebe 1 und 51 zu ermöglichen, verlaufen die Seitenwände 5, 7, 9 und 11 nicht senkrecht zu der Bodenfläche 3 der Siebe 1 und 51. Der Winkel α zwischen den Seitenwänden 5, 7, 9 und 11 und der Bodenfläche 3 beträgt etwas mehr als 90°, vorzugsweise etwa 100 bis 120°. Wie in Fig. 6 dargestellt ist vorgesehen, dass der Griff 39 auf der Oberkante des Siebes 1 bzw. auf den Rahmenelementen 15 und 17 des Siebes 1 zum Aufliegen kommt. Hierdurch wird eine definierte Auflagefläche gebildet und ein zu tiefes Ineinander bewegen, welches ein Verklemmen der beiden Siebe 1 und 51 zur Folge haben kann, vermieden.

Wie aus Figur 7 zu entnehmen ist, sind die beiden Siebe 1 und 51 auch quer zueinander aufeinander stapelbar. Dabei liegt das Bodenrahmenelement 41 des oberen Siebes 51 auf der Oberseite der beiden Rahmenelemente 15 und 17 des unteren Siebes 1 auf. Um eine Verschiebbarkeit des oberen Siebes 51 entlang dessen Längsachse zu vermeiden, ist der Abstand a der beiden Verstrebungen 43 geringfügig größer gewählt, als der Abstand b zwischen den außen liegenden Rahmenelementen 17 in Querrichtung des Siebes 1. Hierdurch wird ein sicheres Stapeln von mehreren Sieben 1 und 51 quer zueinander und aufeinander möglich.

## Patentansprüche

1. Sieb (1, 51), insbesondere Sterilisationssieb, mit einem Boden (3) und mit an dem Boden angeordneten Seitenwänden (5, 7, 9, 11), wobei der Boden (3) und/oder die Seitenwände (5, 7, 9, 11) wenigstens abschnittsweise aus Siebmaterial (13) gebildet sind, **dadurch gekennzeichnet, dass** das Sieb (1, 51) um die Oberkante der Seitenwände (5, 7, 9, 11) zwei weitgehend parallel nebeneinander verlaufende Rahmenelemente (15, 17), welche Drahtelemente sind, aufweist, wobei wenigstens eines der Rahmenelemente (17) definierte Vertiefungen (25, 35) und/oder Erhebungen (21, 31) der Art aufweist, dass ein weiteres derartiges Sieb (51) mit nach oben gerichtetem Boden (3) mit dessen entsprechend ausgebildeten Rahmenelementen (15, 17) auf den Rahmenelementen (15, 17) des ersten Siebes (1) bündig zum Aufliegen kommen kann.

2. Sieb (1, 51) nach Anspruch 1, **dadurch gekennzeichnet, dass** sich die Vertiefungen (25, 35) und Erhebungen (21, 31) über die Eckbereiche (23, 33 und 19, 29) der Seitenwände (5, 7, 9, 11) erstrecken.

3. Sieb (1, 51) nach Anspruch 1 oder 2, **dadurch gekennzeichnet, dass** an den jeweils gegenüberliegenden Eckbereichen (19, 29 und 23, 33) entweder Erhebungen (21, 31) oder Vertiefungen (25, 35) vorhanden sind.

4. Sieb (1, 51) nach Anspruch 2 oder 3, **dadurch gekennzeichnet, dass** die Eckbereiche (19, 29 und 23, 33) zwischen den jeweils an die Eckbereiche angrenzenden Stirn- und Längswänden liegen.

5. Sieb (1, 51) nach einem der vorhergehenden Ansprüche, **dadurch gekennzeichnet, dass** lediglich eines der beiden Rahmenelemente, insbesondere das äußere Rahmenelement (17), Erhebungen (21, 31) oder Vertiefungen (25, 35) aufweist und dass das andere Rahmenelement, insbesondere das innere Rahmenelement (15), keine Erhebungen oder Vertiefungen aufweist.

6. Sieb (1, 51) nach einem der vorhergehenden Ansprüche, **dadurch gekennzeichnet, dass** das Sieb (1) Verbindungsmittel zum Verbinden eines auf dem Sieb (1) aufliegenden, weiteren Siebes (51) vorsieht.

7. Sieb (1, 51) nach einem der vorhergehenden Ansprüche, **dadurch gekennzeichnet, dass** der Boden (3) als auch die Seitenwände (5, 7, 9, 11) von einem Drahtgeflecht (13) gebildet werden, wobei freie Enden (47) der das Drahtgeflecht (13) bildenden Drähte zwischen den beiden Rahmenelementen (15, 17) enden.

8. Sieb (1, 51) nach einem der vorhergehenden Ansprüche, **dadurch gekennzeichnet, dass** an den Seitenwänden (5, 7, 9, 11) ein weitgehend parallel zu dem Boden verlaufendes Mittelrahmenelement (37) vorhanden ist.

9. Sieb (1, 51) nach Anspruch 8, **dadurch gekennzeichnet, dass** das Mittelrahmenelement (37) insbesondere zwei gegenüberliegende Ausformungen (39) aufweist, die als Griffe Verwendung finden können.

10. Sieb (1, 51) nach Anspruch 9, **dadurch gekennzeichnet, dass** wenigstens zwei Siebe (1, 51) derart ineinander stapelbar sind, dass die Ausformungen (39) des Mittelrahmenelements (37) des oberen Siebes (51) auf der Oberseite der Rahmenelemente (15, 17) des unteren Siebes (1) zum Aufliegen kommen.

11. Sieb (1, 51) nach einem der vorhergehenden Ansprüche, **dadurch gekennzeichnet, dass** das Sieb (1, 51) ein auf der Unterseite des Bodens (3) angeordnetes Bodenrahmenelement (41) aufweist, welches der der Oberkante der Seitenwände (5, 7, 9, 11) abgewandte Ausformungen (45) aufweist.

12. Sieb (1, 51) nach Anspruch 11, **dadurch gekennzeichnet, dass** das Bodenrahmenelement (41) Querverstrebungen (43) aufweist, an welchen die Ausformungen (45) angeordnet sind.

13. Sieb (1, 51) nach Anspruch 12, **dadurch gekennzeichnet, dass** wenigstens zwei Siebe (1, 51) quer zueinander aufeinander stapelbar sind, wobei das Bodenrahmenelement (3) des oberen Siebes (51) auf den Rahmenelementen (15, 17) des unteren Siebes (1) aufliegt und die Ausformungen (45) an dem Bodenrahmenelement (41) des oberen Siebes (51) eine Verschiebbarkeit des oberen Siebes (51) in Querrichtung verhindern.

14. Sieb (1, 51) nach einem der vorhergehenden Ansprüche, **dadurch gekennzeichnet, dass** das Sieb (1, 51) vollständig aus Edelstahl ist.

## Claims

1. Screen (1, 51), particularly a sterilisation filter, with a base (3) and with side walls (5, 7, 9, 11) affixed to the base, wherein the base (3) and/or the side walls (5, 7, 9, 11) are formed from filter material (13) at least in sections, **characterised in that** the upper edge of the side walls (5, 7, 9, 11) features two substantially parallel frame elements (15, 17) which are constituted by wire members, whereby at least one of the frame elements (17) exhibits depressions (25, 35) and/or elevations (21, 31) of such a kind that a further suchlike screen (51) with upwardly inclined base (3) can come to rest on the frame elements (15, 17) of the first screen (1) with its correspondingly shaped frame elements (15, 17) so as to form a flush fitting.

2. Screen (1, 51) according to claim 1, **characterised in that** the depressions (25, 35) and elevations (21, 31) extend over the corner sections (23, 33 and 19, 29) of the side walls (5, 7, 9, 11).

3. Screen (1, 51) according to claims 1 or 2, **characterised in that** either elevations (21, 31) or depressions (25, 35) are present at the respectively opposite corner sections (19, 29 and 23, 33).

4. Screen (1, 51) according to claim 2 or 3, **characterised in that** the corner sections (19, 29 and 23, 33) lie between the front walls and the longitudinal walls adjoining the corner sections.

5. Screen (1, 51) according to any one of the preceding claims, **characterised in that** only one of the two frame elements, in particular the outer frame element (17), exhibits elevations (21, 31) or depressions (25, 35) and that the other frame element, in particular the inner frame element (15), does not exhibit any elevations or depressions.

6. Screen (1, 51) according to any one of the preceding claims, **characterised in that** the screen (1) is provided with connecting means to connect an additional screen (51) lying on the screen (1).

7. Screen (1, 51) according to any one of the preceding claims, **characterised in that** the base (3) as well as the side walls (5, 7, 9, 11) consist of wire netting (13), wherein free ends (47) of the wire constituting the wire netting (13) terminate between the two frame elements (15, 17).

8. Screen (1, 51) according to any one of the preceding claims, **characterised in that** a mid-frame element (37) is provided on the side walls (5, 7, 9, 11), which runs substantially parallel to the base.

9. Screen (1, 51) according to claim 8, **characterised in that** the mid-frame element (37) features two opposite mouldings (39) which can be used as grips.

10. Screen (1, 51) according to claim 9, **characterised in that** at least two screens (1, 51) are stackable, one within the other, in such a manner that the mouldings (39) of the mid-frame element (37) of the upper screen (51) come to rest on the upper side of the frame elements (15, 17) of the lower screen (1).

11. Screen (1, 51) according to any one of the preceding claims, **characterised in that** the screen (1, 51) has a base frame element (41) on the lower side of the base (3), which exhibits mouldings (45) that are turned away from the upper edge of the side walls (5, 7, 9, 11).

12. Screen (1, 51) according to claim 11, **characterised in that** the base frame element (41) features crossbars (43) on which the mouldings (45) are positioned.

13. Screen (1, 51) according to claim 12, **characterised in that** at least two screens (1, 51) are stackable crosswise onto one another, whereby the base frame element (3) of the upper screen (51) rests on the frame members (15, 17) of the lower screen (1) and the mouldings (45) on the base frame element (41) of the upper screen (51) prevent slippage of the upper screen (51) in the transverse direction.

14. Screen (1, 51) according to any one of the preceding claims, **characterised in that** the screen (1, 51) is made in its entirety of stainless steel.

## Revendications

1. Tamis (1, 51), en particulier tamis destiné à la stérilisation, avec un fond (3) et des parois latérales (5, 7, 9, 11) disposées sur le fond, moyennant quoi le fond (3) et / ou les parois latérales (5, 7, 9, 11) sont au moins en partie constituées de matériau filtrant (13), **caractérisé en ce que** le tamis (1, 51) présente, autour du bord supérieur des parois latérales (5, 7, 9, 11), deux éléments de cadre (15, 17) disposés l'un à côté de l'autre en étant sensiblement parallèles, qui sont des éléments en fil métallique, moyennant quoi au moins l'un des éléments de cadre (17) présente des évidements (25, 35) et / ou des saillies (21, 31) défini(e)s, de telle sorte qu'un autre tamis de ce type (51) avec son fond orienté vers le haut (3), avec ses éléments de cadre (15, 17) configurés de manière correspondante, peut reposer à fleur sur les éléments de cadre (15, 17) du premier tamis (1).

2. Tamis (1, 51) selon la revendication 1, **caractérisé en ce que** les évidements (25, 35) et saillies (21, 31) s'étendent sur les zones angulaires (23, 33 et 19, 29) des parois latérales (5, 7, 9, 11).

3. Tamis (1, 51) selon l'une quelconque des revendications 1 ou 2, **caractérisé en ce que**, au niveau de chacune des zones angulaires (19, 29 et 23, 33) respectivement opposées l'une à l'autre, il existe soit des saillies (21, 31) soit des évidements (25, 35).

4. Tamis (1, 51) selon l'une quelconque des revendications 2 ou 3, **caractérisé en ce que** les zones angulaires (19, 29 et 23, 33) se trouvent entre les parois frontales et longitudinales respectivement adjacentes aux zones angulaires.

5. Tamis (1, 51) selon l'une quelconque des revendications précédentes, **caractérisé en ce que** seul l'un des deux éléments de cadre, en particulier l'élément de cadre extérieur (17), présente des saillies (21, 31) ou évidements (25, 35), et **en ce que** l'autre élément de cadre, en particulier l'élément de cadre intérieur (15), ne présente pas de saillies ou d'évidements.

6. Tamis (1, 51) selon l'une quelconque des revendications précédentes, **caractérisé en ce que** le tamis (1) présente des moyens de raccordement permettant le raccordement avec un autre tamis (51) reposant sur le tamis (1).

7. Tamis (1, 51) selon l'une quelconque des revendications précédentes, **caractérisé en ce que** le fond (3) ainsi que les parois latérales (5, 7, 9, 11) sont constitués d'un grillage en fil métallique (13), moyennant quoi les extrémités libres (47) des fils métalliques formant le grillage en fil métallique (13) débouchent entre les deux éléments de cadre (15, 17).

8. Tamis (1, 51) selon l'une quelconque des revendications précédentes, **caractérisé en ce que**, sur les parois latérales (5, 7, 9, 11), il existe un élément de cadre central (37) sensiblement parallèle au fond.

9. Tamis (1, 51) selon la revendication 8, **caractérisé en ce que** l'élément de cadre central (37) présente en particulier deux déformations (39) se trouvant en face l'une de l'autre, qui sont utilisées sous la forme d'éléments de préhension.

10. Tamis (1, 51) selon la revendication 9, **caractérisé en ce qu'**au moins deux tamis (1, 51) peuvent être empilés les uns dans les autres, de telle sorte que les déformations (39) de l'élément de cadre central (37) du tamis supérieur (51) sont amenées à reposer sur le côté supérieur des éléments de cadre (15, 17) du côté inférieur (1).

11. Tamis (1, 51) selon l'une quelconque des revendications précédentes, **caractérisé en ce que** le tamis (1, 51) présente un élément de cadre du fond (41) disposé sur le côté inférieur du fond (3), qui présente des déformations opposées au bord supérieur des parois latérales (5, 7, 9, 11).

12. Tamis (1, 51) selon la revendications 11, **caractérisé en ce que** l'élément de cadre du fond (41) présente des entretoises transversales (43) sur lesquelles sont disposées les déformations (45).

13. Tamis (1, 51) selon la revendication 12, **caractérisé en ce que** deux tamis (1, 51) peuvent être empilés l'un sur l'autre transversalement, moyennant quoi l'élément de cadre du fond (3) du côté supérieur (51) repose sur les éléments de cadre (15, 17) du tamis inférieur (1), et les déformations (45) sur l'élément de cadre du fond (41) du tamis supérieur (51) empêchent le tamis supérieur (51) de se déplacer dans la direction transversale.

14. Tamis (1, 51) selon l'une quelconque des revendications précédentes, **caractérisé en ce qu'**il est complètement réalisé en acier spécial.
